(19) **Europäisches Patentamt**

**European Patent Office**

**Office européen des brevets**

(11) **EP 1 027 890 A2**

(12) **EUROPEAN PATENT APPLICATION**

(43) Date of publication:
**16.08.2000 Bulletin 2000/33**

(21) Application number: **98123943.7**

(22) Date of filing: **16.12.1998**

(51) Int. Cl.⁷: **A61K 31/00**, A61K 31/4465,
C12N 15/63, C07K 14/47,
G01N 33/53, A61P 25/18,
A61P 35/00

(84) Designated Contracting States:
**AT BE CH CY DE DK ES FI FR GB GR IE IT LI LU
MC NL PT SE**
Designated Extension States:
**AL LT LV MK RO SI**

(71) Applicant:
**KNOLL AKTIENGESELLSCHAFT
67061 Ludwigshafen (DE)**

(72) Inventors:
• **Garcia-Ladona, Francisco Javier, Dr.
76870 Kandel (DE)**
• **Lang, Sandra
67063 Ludwigshafen (DE)**

(74) Representative:
**Bieberbach, Andreas, Dr. et al
BASF Aktiengesellschaft,
Patentabteilung ZDX/G C 100
67056 Ludwigshafen (DE)**

(54) **Homer a new target of treating psychiatric disorders**

(57) A method for treatment of psychosis, schizophrenia, oncological disorders, tumors and/or CNS disorders in a human being comprising administering to said human being a composition comprising an effective amount of a compound which interacts with homer or metabotropic receptors are disclosed.

EP 1 027 890 A2

Printed by Xerox (UK) Business Services
2.16.7 (HRS)/3.6

**Description**

Schizophrenia is a chronic psychiatric disorder affecting approximately 1% of the adult population. The economic and social cost of schizophrenia and other psychotic disorders are considerable due to the large index of hospitalization. The real causes of schizophrenia remains still unknown. The symptoms, classified as positive (hallucinations) and negative (social withdrawal, paranoia) may be observed in some cases as early as in adolescence. Schizophrenic patients suffer a progressive degradation of mood, though and cognition processes (Wright I. and Woodruff P., 1995). Compounds with a beneficial effect on the treatment of schizophrenia or psychosis have been so-called neuroleptics. Early studies suggested that an alteration of the brain dopaminergic system may be related to schizophrenic and psychotic symptoms. Although the alteration of dopaminergic function in the brain of schizophrenics is evident, whether the onset of the disease is due to this alteration or it is only a delayed consequence of the disorder remains unknown. An intense research has been developed regarding the brain dopaminergic system and the pharmacology of dopamine receptors. Typical antipsychotics, such haloperidol, with a strong therapeutic effect on the treatment of psychosis have high affinity to D2 dopamine receptors (Seeman 1987). However, this property is associated to a high incidence of extrapyramidal side effects in most of the cases in a irreversible form (Gratz S.S. and Simpson G.M. 1994; Ebadi M. and Srinivasan S.K. 1995). In addition, haloperdiol have also high affinity for sigma receptors supporting them as a therapeutic target for the treatment of psychosis (Reynolds G.P. and Czudek C. 1995). Drugs specific for other brain receptors have been also proposed as antipsychotics (Fatemi H. et al., 1996). Atypical antipsychotics with mixed pharmacological profile, like clozapine, has been very useful for a effective and safer treatment of psychosis. However, to date, no fully efficient treatment have been found for the treatment of neither psychosis nor neuroleptic malignant syndrome.

It has been demonstrated that dopamine receptor blockade after acute treatment with neuroleptics induces genomic responses in brain (Deutch A.Y. 1996). In particular, transcription factors such c-fos and c-jun are rapidly overexpressed and translocated to the cell nuclei leading to further genomic regulation processes. The effect seems to be related to antagonism at dopamine D1- and D2-receptors. The genomic response induced by neuroleptics may be involved not only to their beneficial effects of antipsychotics but also to their undesirable side effects.

The knowledge of gene expression changes induced by neuroleptics may help to understand both the beneficial and side effects of antipsychotic drugs and therefore also to define new and more effective targets for the treatment of schizophrenia and psychosis. The aim of invention was to disclose genomic effects induced by the treatment of neurolpetics and subsequently to identify new targets for the treatment of psychotic and neurodegenerative disorders.

Recently it has been shown that gene expression of a synaptic protein (homer) can be up-regulated by different stimulus such treatment with the neurostimulant cocaine, seizures or brain synaptic activity particularly during the development (Brakeman P.R. et al., 1997). Homer protein is new 28 kd synaptic protein which coding gene has been sequenced (Ozawa K.A. et al., 1997; Brakeman P.R. et al., 1997). The amino acid sequence contains a PDZ-domain. Homer protein shares only a 10% homology with other members of the PDZ-family thus establishing a putative new group. Homer protein is able to interact with the intracytoplasmic part of metabotropic glutamate receptor proteins mGluR1A and mGluR5 (Brakeman P.R. et al., 1997). These excitatory aminoacid receptors are couplet to excitotoxic mechanisms in brain (Knöpfel T. and Gasparini F. 1996). The precise role of homer in the central nervous system is not yet elucidated. However, the fact that homer protein contains a PDZ domain strongly suggests, as for other proteins containing such domain, the possibility of interaction with other cellular proteins involved in cell signaling systems (Pointing C.P. and Phillips C., 1995) and not only to metabotropic receptors.

The present invention provides the identification of cell systems useful for the study of homer function and as tools for the discovery of new therapeutic compounds related to this protein. The present invention provides human homer gene sequences.

The present invention provides the evidence that homer gene expression can be up regulated in vivo by the treatment with haloperidol.

The present invention also provides the partial sequence of rat and chinesse hamster homer gene and the evidence that homer protein may also be expressed by astrocytes.

The present invention regards the effects of antipsychotic treatment on homer gene expression and the identification of intervention targets for the treatment of schizophrenia, Tourette's syndrome, obsesive compulsive disorders, and other psychotic disorders in general. The present invention also concerns the identification of homer protein, metabotropic and sigma receptors as targets for the identification and preparation of medicinal compounds useful for the treatment of neurodegenerative processes such as senile dementia of Alzheimer's type, argirophilic grain disease and other senile dementias in general, Parkinson's disease and atypical forms of Parkinson's disease, Huntington's disease, demyelinating diseases such multiple sclerosis, progressive multifocal leukoencephalopaty, infection-induced demyelinization, and demyelinization disorders of genetic origin, amyotrophic lateral sclerosis and HIV induced dementia. The present invention also provides a new target for the treatment of CNS diseases with a evident glial cell reaction. The present invention provides new therapeutic targets for the treatment of leukemia and also brain tumors.

Regulation of homer gene expression by haloperidol

Methods

Animals

Adult Sprague-Dawley rats (250 g) were maintained in normal environmental conditions with free access to food and water ab libitum.

Treatment

Animals were treated with haloperidol (0.5 and 5 mg/kg) or saline. Naive (non-treated) animals were used as additional controls.

Example 1. Tissue preparation.

The animals were sacrificed by decapitation 90 minutes after treatment. Whole brain was rapidly removed from the skull, frozen in dry ice and stored at $-30^{\circ}$C. Rat brain sections (15 μm) were obtained at $-20^{\circ}$C in a cryostat, mounted in gelatine-coated slides and stored at $-30^{\circ}$C until used.

Example 2. Synthesis and labelling of oligonucleotides

Olygonucleotide sequences 40 base length were selected front the homer rat gene sequence published in the GENEBANK (accession number: U92079). Antisense oligonucleotide homerAT (5'-CTCGAGTGCTGAAGATAGGTTGTTCCCCCATTTTG-CCCA-3') was complementary to bases 559 to 599. Antisense oligonucleotide homerBT (5' -GTTCCATCTTCTCCT-GCGACTTCTCCTTTGCCAGCCGAGC-3') was complementary to bases 894-934. The corresponding sense oligonucleotides (homerAS and homerBS) were used as control probes. Synthesis was performed in a 395 Applied Biosystems DNA Synthesizer. Purified oligonucleotides were dissolved in DEPC-treated water and stored at $-30^{\circ}$C until use. Labelling of synthetic oligoprobes was performed using a deoxynucleotidyl-transferase (TdT) labelling kit (NEP-100, NEN, Bad-Homburg, Germany). Briefly, 5 pmol of probe were incubated (2 hr) with TdT (36 units) in presence of 50 pmol of [$^{35}$S] dATP (NEN) and Cl$_2$Co. The reaction was stopped and the labelled oligonucleotides purified by column chromatography. Labelling efficacy was checked by paper chromatography in phosphate buffer system.

Example 3. In situ hybridization histochemistry

Homer gene expression in rat brain was studied using in situ hybridization techniques well known in the art. In situ hybridization was performed as previously described (Garcia-Ladona et al., 1994). Briefly, Tissue sections were fixed with paraformaldehyde in PBS and treated (min) with pronase (0.25 mg/l), rapidly washed, dehydrated by consecutive incubation with 60%, 80%, 90% and 100% ethanol, rapidly dried and used for hybridization. Brain sections were incubated with 100 μl of 10 mM Tris-HCl hybridization buffer (pH: 7.5) containing 50% formamide, 0.6 M NaCl, 1x Denhardt's solution, 1 mM EDTA, 0.58 mg/ml yeast t-RNA, 10% dextran sulphate, 10 mM DTT and [$^{35}$S]-labelled oligoprobe (13500 c.p.m./μl). A nescofilmR strip was deposed over hybridization mixture to avoid evaporation and the slides incubated overnight at $42^{\circ}$C in a humid chamber. Afterwards, hybridization solution was washed out from the slides and non specific hybridization was eliminated by incubation (4x 1 hr) with 10 mM Tris-HCl ph:7.5 containing 0.6 M NaCl and 1 mM EDTA at $60^{\circ}$C. Nucleic acids in the sections were precipitated by two consecutive washes with 70% and 95% ethanol containing 0.3 M ammonium acetate. The slides were dried and exposed with a X-O-mat X-ray film (Kodak). The films were developed after 48 hr. Measurements of autoradiographic images were performed with an image analysis system equipped with SigmaScanpro software (Jandel Scientific).

Example 4. Cell culture methods

The culture of HEL cells was performed using conditions commonly used by the art. Cells were grown in RPMI media containing serum (10%), penicilin (90 unit/ml) and streptomycin (90 mg/ml). Culture conditions were 95% humidity and 5% CO$_2$. Cell population was splited 1/3 every 3 days.

The culture of glioma A-172 cells (ATCC) was performed using conditions commonly used by the art. Cells were grown in medium containing special supplement (DMEM NUT F-12) containing serum (10%), penicilin and streptomycin. Culture conditions were $37^{\circ}$C, 95% humidity and 5% CO$_2$. Cells were grown till confluence and then splited.

The culture of glioma U87 cells (ATCC) was performed using conditions commonly used by the art. Cells were grown in medium containing special supplement (DEMEM NUT F-12) containing serum (10%), penicilin and streptomycin. Culture conditions were $37^{\circ}$C, 95% humidity and 5% CO$_2$. Cells were grown till confluence and then splited.

Example 6. Culture of glial cells

The culture of glial cells was performed by methods clearly described in the art. Whole brains from new born rats were dissected out of the skull in aseptic conditions. Brain areas (striatum, cortex and hippocampus) were dissected and immersed in culture medium without serum. Small pieces of tissue were obtained by scretchin g with micro forceps. Tissue was homogenated by 10 passages through a 1.2 mm gauge. Cell suspensions were centrifugated and pellet resuspended in culture medium and plated in pedri dishes. Cell were incubated for 2-3 hr at 37°C, 95% humidity, 5% $CO_2$. Non-attached cells were aspirated and centrifugated. Pelleted cells were resuspended in medium containing 10% serum and antibiotics and then plated in petri dishes for 10-20 days. Medium was renewed every 3-4 days.

Example 7. RNA isolation

The isolation of RNA from different cell and tissue sources was performed using a single-solution extraction method commercially. available (Trizol®, Gibco Life Sciences). The cells were washed with RNAse free PBS and the homogenized with Trizol®. The total RNA present in each sample was determined by measuring light abosrbance and extrapolating to a calibration curve.

Example 8. RT-PCR methods

Homer gene expression in human glioma cells (A-172, U87), in brain areas, in glial cell cultures, and in HEL cells was studied using reverse transcriptase and polymerase chain reaction methods of common use in the art. A commercially available RT-PCR kit was used (Ready to go, Amersham/Pharmacia Biotec). Protocols were adjusted to the supplier recommendations. RT-PCR was performed using 1 mg total RNA. Primers were selected by analysing the homer gene sequence (accession number: U92079) logged in the GENEBANK. Thermocyler was programmed as follows (RT 30 min at 42°C, PCR 23 cycles of 1 min 95°C, 1 min 60°C, 2 min 72°C). At the end of PCR cycles, samples of PCR mixture were electrophoresed in agarose gels as described by commonly used protocols.

Example 9. Analysis of nucleic acids by gel electrophoresis

Gels for the analysis of RT-PCR products were prepared by melting agorose (1%) in electrophoresis buffer (Current Protocols in Molecular Biology, John Wiley & Sons, 1995) at 60°C. PCR samples were mixed with sample buffer containing and loaded (1µg/lane). Electrophoresis was run 60 min. and separation of fragments was checked by u.v illumination.

Example 10. Analysis of gene sequences

The analysis of gene sequences obtained by RT-PCR was performed by using software commercially available or in the public domain. The sequence identification was performed by homology search using DNASIS software (HITACHI) and software available in the public GENEBANK.

Results

Homer gene expression is up regulated after antipsychotic treatment.

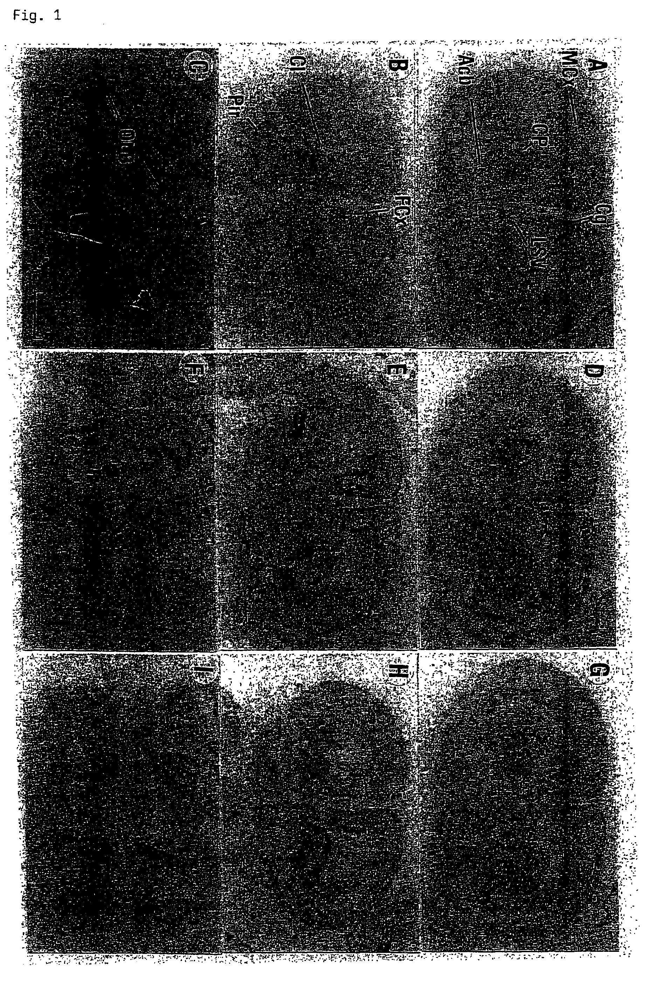

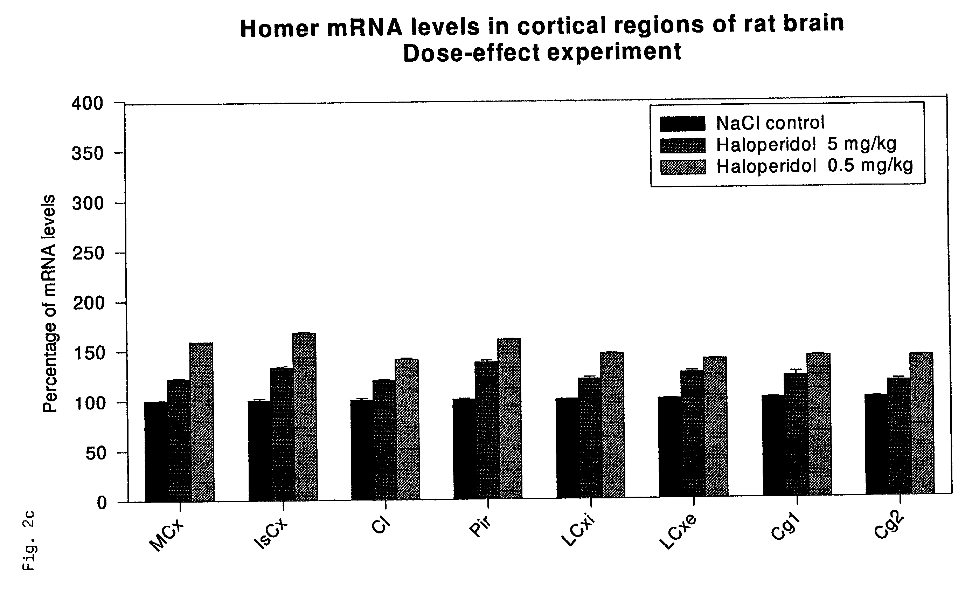

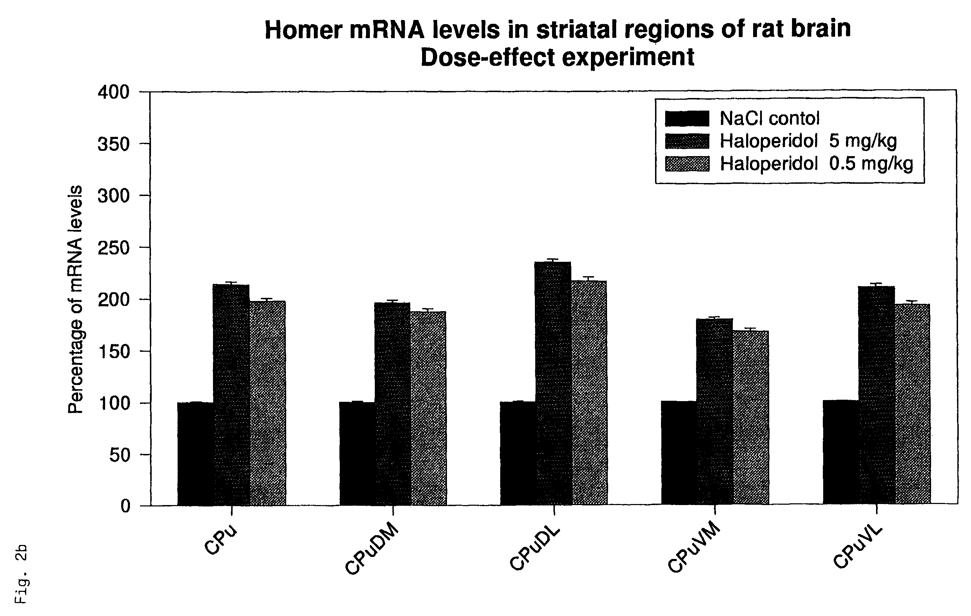

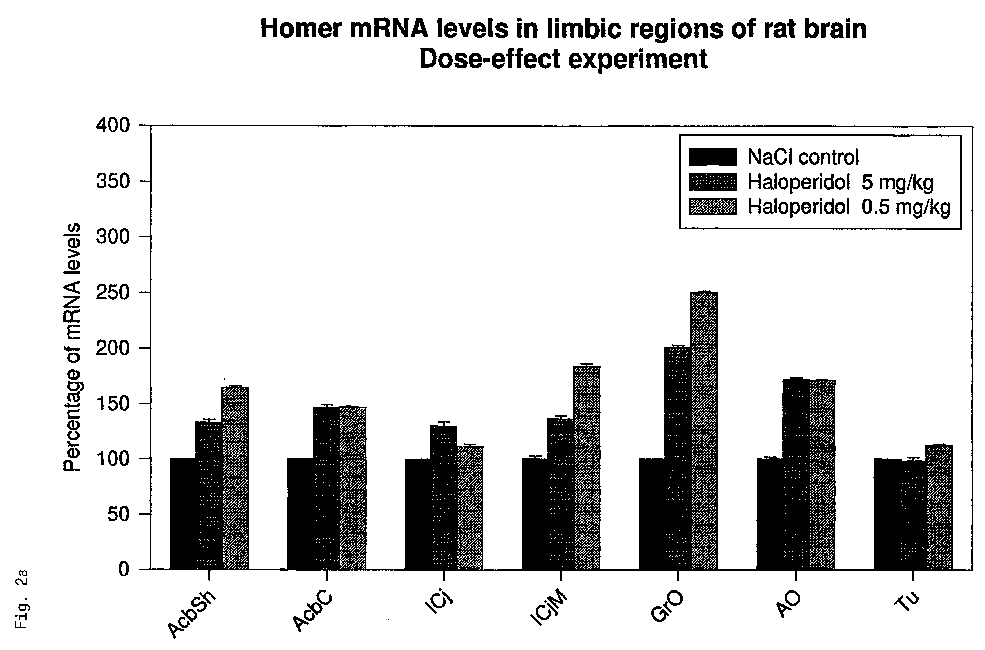

The in situ hybridization images showed that homer mRNA transcritps were present in higher levels in haloperdiol treated animals than in controls (Fig. 1) The differences, on optical density measured in autoradiographic films, between control and treated animals are shown in figure 2.

Homer gene is expressed by neurotumoral cells

The fragments of DNA obtained after RT-PCR using RNA from A-172 and U87 cells and specific primers complementary to homer gene sequences fully agree in their size with the expected values of the homer gene fragments. The sequencing of RT-PCR products demonstrate their identity as sequences located in the homer gene. Homology analysis demonstrate some punctual differences with the rat homer gene sequences (see apendix 1).

Homer gene is expressed by Chinesse Hamster Ovary Cells.

The fragments of DNA obtained after RT-PCR using RNA from CHO cells and specific primers complementary to homer gene sequences fully agree in their size with the expected values of the homer gene fragments. The sequencing of RT-PCR products demonstrate their identity as sequences located in the homer gene. Homology analysis demonstrate some punctual differences with the rat homer gene sequences (see appendix 1).

Homer gene is expressed by human eritroleukemic (HEL) cells.

The fragments of DNA obtained after RT-PCR using RNA from HEL cells and specific primers complementary to homer gene sequences fully agree in their size with the expected values of the homer gene fragments. The sequencing of RT-PCR products demonstrate their identity as sequences located in the homer gene. Homology analysis demonstrate some punctual differences with the rat homer gene sequences (see appendix 1)

Example 11 provides a method to detect the efficacy of antisense oligonucleotides in cultured cells.

Cells were cultured as described in example 4. Cells were treated for different times with different concentrations of antisense, sense and missense oligonucleotides complementary to human homer gene sequences. The presence of homer protein were determined by western blots (example 13) and immunocytochemistry (example 12) using specific antibodies directed against human homer polypeptide sequences. The effects of antisense oligonucleotides complementary to human homer gene sequences were determined by using different methods of to determine second messenger signal pathways activation (see examples 14, 15 and 16)

Example 12 provides a method to detect homer protein by immunocytochemistry. The method was similar to that previously described (Garcia-Ladona 1997)

Example 13 provides a method to detect homer protein by using western blots. The method was similar to that described previously (Garcia-Ladona 1997)

Example 14 provides a method to determine the agonist-induced phospholipase C activity.

The method was basically described previously (Garcia-Ladona et al., 1993). Cells were incubated for 24 hr with 0.125 μM [$^3$H]myoinositol. Non incorporated [$^3$H]myo-inositol was eliminated from the medium and replaced with Krebs-Henseleit buffer containing 10 mM LiCl. After 10 min incubation, different agonist were added for 45 min. The reaction was stopped by replacing the stimulation medium with distilled water. In the case of tissue samples, the procedure is very similar (Garcia-Ladona et al., 1993) Cells were frozen and stored at -80°C. Production of [$^3$H]myo-inositol monophosphate was determined in cell samples by known methods of chromatographic purification (in Methods in Neutrotransmission receptor analysis. Eds H.I. Yamamura, S.J. Enna M.J. Kuhar, Raven Press, 1990). A similar method to determine agonist-mediated phospholipase C stimulation was used by preparing membrane fractions and incubating with [$^{32}$P]PIP$_2$ and agonist or antagonists. In this case the production of IP3 was determined. Methods known of the art have been also optimized for using microtiterplates based systems. Commercially available materials allows to perform high throughput and secondary screening (Amersham pharmacia biotech and NEN).

Example 14 provides a method to determine agonist-induced elevation of intracellular Ca$^{++}$ levels.

The method used was similar to known methods described in the literature (Nuccitelli R, 1994). Briefly cells were grown in culture bottles as indicated (example 4). Cells were softly scraped before reaching confluence. Cell were labelled with Fura-2 by incubating (30 min) with Fura-2-acetyl-methylester at room temperature. Cells were centrifuged at 180 x g for 10 min and resuspended in DMEM-F 12 medium without serum and incubated at 37°C, 5% CO$_2$ and 95% humidity for 45 min. Intracellular calcium levels were determined in a fluorescence microscope equipped with an appropriate filter exchange system ( Plympus / Hamamtsu ). Fluorescence ratio ( A340 / A380 ) was measured using Argus$^®$ software. Intracellular calcium levels were monitored in single cells for a short period in the absence of drugs and then for 30 min after the addition of selected compounds.

Example 16 provides a method to measure agonist-induced cAMP production in cultured cells.

The method was similar to that used currently in the art (In Methods in Neurotransmission receptor analysis. Eds H.I. Yamamura, S.J. Enna, M.J. Kuhar, Raven Press, 1990). Briefly, cells were incubated for 10 min in culture medium in the absence of serum and antibiotics. Reaction was stopped by heating to 95°C 15 min. Cell samples were frozen and stored at -80°C. cAMP levels were determined with commercial available kits (Biotrak from Amersham) using the cAMP binding protein. Methods known of the art have been also optimized for using microtiterplates based systems. Commercially available materials allows to perform high throuphput and secondary screening (Amersham and NEN).

Example 17 provides a method to determine the effects of antisense oligonucleotides in vivo.

Antisense nucleotides were dissolved in physiological saline and injected intravenously and intracerebroventricularly in animals. Different periods of treatment were established. After treatment, animals were sacrificed and different organs and body fluids used for histological analysis. Animals were used to determine the effects on the homer and other cellular proteins production by using immunohistochemical, immunocytochemical and western blot methods described in the examples 12 and 13 respectively. Animals were also used to determine the effect on cell signalling processes by methods exemplified in the examples 14 and 16. A group of animals were tested in behavioural models for antipsychotic effects (see examples).

Example 18 provides a method to determine the efficacy of a compound in an animal model of psychotic disorder.

The method have been reported in the literature by injecting PCP in animals. Animals were treated before and after PCP with different doses of compound. Afterwards a set of animals were used in behavioural models predictive of psychotic activity to asses compound's efficacy (example 28). An additional set of animals may be used for the analysis of different receptor activity as described in example 14 and 17.

Example 19 provides a method to determine the efficacy of compounds on preventing neuroleptic induced malignant syndrome.

Different animals models are may be used including haloperidol induced catalepsy and chronic treatment with haloperidol. Animals may be subsequently used to determine behavioural deficiencies (example 28, anatomical neurodegeneration and changes in gene expression (as exemplified in examples 1-3, 12, 13).

Example 20 provides a method to determine the efficacy of a compound in the treatment of demyelinating diseases. Different animals models of demyelinization are known of the art. Demyelinization was induced by injecting antibodies. Animals were treated with the compounds after the induction of myelin loss. Animal brains were used to determine the levels and integrity of myelin.

Example 21 provides a method to determine the efficacy of a compound in the treatment of demyelinizating diseases.

The method consist in the use of oligodendrocytes-enriched cell cultures from normal and demyelinizated animals (jimpy mutation) as described (Garcia-Ladona et al., 1997). Cells were treated with different doses of the compound and the integrity of myelin sheets and the levels of myelin markers were determined.

Example 22 provides a method to determine efficacy of a compound in Parkinson's disease. Different models were used, MTP induced Parkinsonism in mice, 6-OHDA induced degeneration in substantia nigra (Drug Discovery and Evaluation, Eds. H.G. Vogel and W.H. Vogel 1997).

Example 23 provides a method to determine the beneficial effects of a compound in senile dememntia of Alzheimer type.

The method is known of the art and consists on the use of transgenic animals overproducing b-amyloid protein. Animals can be treated with compounds and analysed for memory deficits and other behavioural parameters.

Examples 24 provides a method to identify compounds with high affinity to metabotropic receptors. Similar methods have been extensively described in the literature (Drug Discovery and Evaluation, Eds. H.G. Vogel and W.H. Vogel 1997).

Binding saturation kinetics of a radioligand. Membranes (200 $\mu$l) were incubated (600 $\mu$l total volume) in 100 mM Tris-HCl (pH: 7.7) containing 1 mM EDTA (buffer B) with increasing concentrations of radioligand in the presence (non specific binding) or in the absence (total binding) of an antagonist at high concentration. Incubation was prolonged for 90 min at 30°C; afterwards, samples were filtered, using a Skatron filtration system, through GF/B filters embedded in 0.3% poly-ethylenimide. Filters were washed with 9 ml of butter B at 4°C. Radioactivity retained in the filters was measured by liquid scintillation counting using 5 ml Ultima-Gold.

Displacement of radioligand binding: Binding displacement experiments were performed basically as reported in other studies. Membranes (200 $\mu$l) were incubated in buffer B (600 $\mu$l total volume) with increasing concentrations of the selected compounds in the presence of a selected concentration of radioligand. After a 87 min incubation period at 30°C, samples were filtered with buffer B at 4°C through GF/B filters. Filters were washed with 9 ml buffer B. Radioactivity retained in the filters was determined as above. Total binding was defined as radioligand binding observed in the absence of other compounds. Non specific binding was defined as radioligand binding levels observed in the presence of antagonist in high concentration.

Analysis of radioligand binding data Saturation parameters radioligand were estimated both by no-linear regression analysis and from linear plots by using SigmaPLot software (Jandel Scientifivc Germany). Displacement curves were build from radioactive binding levels expressed as percentage of total binding. $IC_{50}$ and Hill coefficients ($n_H$) were estimated by non linear regression analysis.

Example 25 provides a method to identify compounds with agonist activity at different receptors by measuring agonist stimulated [$^{35}$S]GTP$_\gamma$S binding.

The methods are very well known in the art (Hilf and Jakobs 1992). Briefly, agonist activity was determined by measuring drug-induced changes of [$^{35}$S]GTP$_\gamma$S binding in membranes from cells. Cell membranes were obtained as indicated above. [$^{35}$S]GTP$_\gamma$S binding assay was performed using a previously described method with minor modifications. Cell membranes (12 $\mu$g) were incubated with 50 mM trietanolamine-HCl buffer (pH: 7.5) containing 6.75 mM MgCl$_2$, 150 mM NaCl, 1 mM DTT, 1 mM EDTA, 10 $\mu$M GDP and [$^{35}$S]GTP$_\gamma$S (nM). Following 60 min incubation, at 30°C, in the absence or in the presence of different drug concentrations, the assay mixture (100 $\mu$l) was rapidly filtrated through GF/B filters using a Skatron**®** filtration device. Filters were quickly washed with 9 ml of 50 mM Tris-HCl buffer (4°C) containing 100 mM NaCl, 5 mM MgCl$_2$ at pH: 7.5. Radioactivity retained in the filters was determined by scintillation spectrometry using Ultima-Gold scintillation liquid. Drug activities were expressed as % of basal binding levels measured in the absence of the compound. Curves were fitted using a non-linear regression analysis software (Sigma Plot, Jandel Scientific, GErmany) to the general equation $E = (L \cdot E_{max})/(L + EC_{50})$ where E is the effect, L is ligand concentration, $E_{max}$ is the maximal effect and $EC_{50}$ is the concentration inducing 50% of the maximal effect.

Example 26 provides a method to prepare cell membranes.

Different methods have been described in the art (Biologiacal Membranes, Eds). Membranes were prepared form cell cultures. Cells were softly scraped from bottle surface and centrifuged 10 min at 180 x g. Cell pellets were resuspended in 5 mM Tris HCl buffer (pH: 7.6), containing 5 mM EDTA, 5 mM EGTA, 0.1 mM PMSF and 3 mM benzamidine (buffer A) and incubated for 15 min at 4°C. Cell suspension was homogenized (6 x 3s) in an Ultraturrax (15000 r.p.m.) and centrifuged for 1 min at 1000 x g and 4°C. Nuclear pellet was resuspended in buffer A, homogenized and centrifuged as above. Supernatants of both steps were collected and centrifuged for 20 min at 40000 x g at 4°C; pellet was resuspended in buffer A and homogenized (1 x 15s). Membrane suspension was centrifuged for 20 min at 40000 x g at 4°C. The resulting pellet was resuspended in buffer A containing 10% glycerol and 1% bovine serum albumin. Aliquots were frozen and stored at -80°C until use.

Example 28 provides a method to study psychosis by using animal models.

The method has been described in the literature (Swerdlow N.R. et al., 1996). Animals may be treated by different routes with compounds and checked for behavioural parameters to determine efficacy as antipsychotic agents (Drug Discovery and Evaluation, Eds H.G. Vogel and W.H. Vogel, 1997).

Example 29 provides a method to study psychosis by using a neonatal lesion.

The method is very weel known of the art (Lipska et al., 1993). Animals are lesioned with a excitotoxin in the central hippocampal area in the neonatal period and are used in the adulthood. Animals may be treated with compounds and checked for different behavioural parameters including prepulse inhibition paradigm (example 28).

The term human homer gene refers to polynucleotide sequences with homology to the so-called rat homer gene. The term human homer protein refers to a peptide resulting of the translation of human homer gene sequences using the natural code.

The present invention provides four partial nucleotide sequences of human homer gene.

The present invention also provides partial nucleotide sequences of homer gene of hamster and homer protein expressed by astrocytes in culture.

Nucleic acid sequences according with the present invention can be used to design anti-sense oligonucleotides and to determine aminoacid sequences of polypeptides encoded by them.

Modified antisense oligonucleotide synthesis is well known in the art (Gene Therapy, Eds, J.T. August, 1997). Different oligonucleotide modifying groups can be used. Modified anti sense oligonucleotides will be tested to determine time-life, bioavailability an efficacy on inhibiting the homer protein translation (examples 11, 12 and 13).

The human homer peptides of present invention can be used to raise specific antibodies. The present invention also includes the use of antibodies or antisense oligonucleotides raised against human homer protein as therapeutic compounds and as probes to detect human homer protein and gene respectively (see examples 1, 3, 12 and 13). Where probes means unlabelled or isotope or non-isotopically labelled compounds that bind to a specific target. The antibodies against the human homer sequence can be obtained using the known protein chemistry techniques.

The present invention also includes a method to disclose the role of homer protein in neurotumoral and leukemic cells for example in invasive activity, proliferation, cell survival, apoptosis, signal transduction, genomic activity toxicity, sensibility to infectious agents and biological and chemical compounds.

The present invention includes a method to study the role of homer protein on the activity of other cell signalling mechanisms by using HEL cells and A-172 and U97 human glioma cells.

The present invention includes a method to study the role of human homer protein on the activity of specific cell proteins and receptors by transfecting their genes in HEL cells, A-172 and U87 human glioma cells and CHO cells. Tranfection techniques are well known of persons skilled in the art and involve the transference into the cell of gene sequences included in a vector. Where vector means polynucleotide sequences that facilitate the insertion in a host of a given genetic information. Where vectors include plasmids and eucaryotic viruses and bacteriophages. Many vectors and expression systems are well known and documented in the art (for example pcDNA3, pCR2.1 from Invitrogene).

Methods to detect human homer

The present invention includes a method to detect human homer protein in human brain by using antisense oligonucleotides or antibodies indicated above. Examples of such methods are reported in examples 1, 2, 3, 12 and 13).

The present invention provides a method to detect human brain (glial) tumors by using antibodies directed against human sequences of homer or using isotope- or non-isotopically-labelled oligonucleotide probes complementary to human homer sequence. Methods are exemplified in examples 1, 2, 3, 12 and 13.

The present invention includes a method to detect human glioblastome and leukemic cells in culture using antibodies directed to human homer protein or antisense nucleotides complementary to human homer protein gene sequences. Methods for such detection are reported in examples 2, 3, 4, 12 and 13.

Neurodegenration and homer

The present invention includes a method to treat human brain degenerative processes by using compounds increasing (modifying) homer gene expression. Methods to identify such compounds are exemplified in the examples.

Where degenerative processes are ischemia of vascular origin, ischemic states induced by brain or spinal cord trauma, epilepsy, psychotic disorders including schizophrenia, senile dementia including senile dementia of Alzheimer's type, demielynating diseases, HIV induced dementia and neurodegeneration involving excitatory aminoacid receptors and neurodegeneration involving reactive glial cells. Degenerative processes is a term used also as synonym of neurodegeneration and of neurodegenerative disease.

The present invention also provides a method for treating or prevent neurodegeneration by using compounds facilitating (modifiying) the interaction between homer and other components of the cell signalling pathways, genetic information or cellular proteins. Where genetic information is any DNA or RNA sequences present in the cell.

The present invention also provides a method for treating or prevent neurodegeneration by using compounds facilitating the interaction between homer and metabotropic receptors. Such compounds may be identified by coincubating membranes from cells expressing metabotropic receptors, purified human homer protein, antibodies directed to homer protein and by using a commercial methods (SPA, Amersham or flashplates) to detect binding activities.

The present invention also provides a method for treating or prevent neurological deficits observed in patients suffering of neurodegenerative diseases by using agonist/antagonist of metabotropic receptors. Such compounds may be identified using current membrane binding methods as described in example ...

The present invention also provides a method for treating or prevent neurological deficits observed in patients suffering of squizophrenia or any other psychotic disorders by using agonist/antagonist of metabotropic receptors. Such compounds will be identified using membrane binding methods described in the examples.

The present invention also contains a method to treat and prevent neurological deficits induced after treatment with typical antipsychotics by using antagonist/agonist of metabotropic receptors. Such compounds will be identified using membrane binding methods described in the examples.

The present invention contains a method to modify the expression of homer protein by using compounds with affinity to sigma or dopaminergic receptors. The compounds will be selected by using in the examples. Their efficacy will be determined by using the methods described in examples 1, 2, 3, 6, 7, 8 and 9.

Brain tumors and leukemias

The present invention provides a method to treat human brain (glial) tumors by using modified or unmodified antisense oligonucleotides complementary to human homer mRNA sequences or by using antibodies directed against human homer protein, or by using compounds modifying the expression of homer protein acting directly in the transcription or in the translation, protein folding, protein maturation, protein turnover processes, or by using compounds that modify the interaction between homer and any other cellular protein or peptide included those involved in signalling processes, and genetic information. Where genetic information is DNA or RNA sequences. Compounds may active by different treatments including intravenous application, orally treatment or stereotaxically injected in the brain tumor area.

The present invention provides a method to treat leukemias by using modified or unmodified antisense oligonucleotides complementary to human homer mRNA sequences or by using antibodies directed against human homer protein, or by using compounds modifying the expression of homer protein acting directly in the transcription or in the translation, protein folding, protein maturation, protein turnover processes, or by using compounds that modify the interaction between homer and any other cellular protein or peptide included those involved in signalling processes, and genetic information. Where genetic information is DNA or RNA sequences. Compounds may active by different treatments including intravenous application and oral treatment.

Sigma compounds and homer.

The present invention also provides a method to modify (block /stimulate) the effects of sigma selective compounds by antisense oligonucleotides or antibodies directed to human homer protein. The effectivity of such treatments can be determined by studying their effects on different animal species. Animals will be treated with as in the example. Afterwards, animals will be observed in different behaviour and biochemical paradigms to determine the blockade or the potentiation of sigma receptor compound activity.

The present invention also provides a method to modify (block /stimulate) the effects of sigma selective compounds. By using antagonist/agonist of metabotropic receptors. Such compounds can be selected by studying their effects on different animal species. Animals will be treated with particular compounds selected for their activity at metabotropic receptors (see method in the examples). Afterwards, animals will be observed in different behaviour and biochemical paradigms to determine the blockade or the potentiation of sigma receptor compounds.

The present invention also provides a method to determine the in vivo and in vitro effects of sigma receptor selective compounds. Their efficacy will be determined by using the methods described in examples 1, 2, 3, 6, 7, 8 and 9.

The present invention also provides a method to determine the in vivo and in vitro effects of typical and atypical antipsychotics. The compounds will be selected by using examples ... Their efficacy will be determined by using the methods described in examples 1, 2, 3, 6, 7, 8 and 9.

Appendix 1
Sequence of homer gene amplified from HEL mRNA and its correspondign
aminoacid sequence

A CTCGAGCTCA TGTCTTCCAA ATTGACCCAA ACACAAAGAA GAACTGGGTA
CCCACCAGCA AGCATGCAGT TACTGTGTCT TATTTCTATG ACAGCACAAG AAATGTGTAT
AGGATAATCA GTTTAGATGG CTCAAAGGCA ATAATAAATA GTACCATCAC CCCAAACATG
ACATTTACTA AAACATCTCA GAAGTTTGGC CAGTGGGCTG ATAGCCGGGC AAACACCGTT
TATGGATTGG GATTCTCCTC TGAGCATCAT CTTTCGAAAT TTGCAGAAAA GTTTCAGGAA
TTTAAAGAAG CTGCTCGACT AGCAAAGGAA AAATCACAAG AGAAGATGGA ACTTACCAGT
ACACCTTCAC AGGAATCCGC AGGCGGGGAT CTTCAGTCTC CTTTAACACC GAAAGTA


STRAHVFQID PNTKKNWVPT SKHAVTVSYF YDSTRNVYRI ISLDGSKAII NSTITPNMTP
TKTSQKFGQW ADSRANTVYG LGFSSEHHLS KFAEKFQEFK EAARLAKEKS QEKMELTSTP
SQESAGGDLQ SPLTPKVXG


Appendix 2 Homer gene sequence amplified from U87 mRNA and its
correspondign aminoacid sequence

A TGGGGGAGCA ACCTATCTTC AGCACTCGAG CTCATGTCTT CCAAATTGAC
CCAAACACAA AGAAGAACTG GGTACCCACC AGCAAGCATG CAGTTACTGT GTCTTATTTC
TATGACAGCA CAAGAAATGT GTATAGGATA ATCAGTTTAG ATGGCTCAAA GGCAATAATA
AATAGTACCA TCACCCCAAA CATGACATTT ACTAAAACAT CTCAGAAGTT TGGCCAGTGG
GCTGATAGCC GGGCAAACAC CGTTTATGGA TTGGGATTCT CCTCTGAGCA TCATCTTTCG
AAATTTGCAG AAAAGTTTCA GGAATTTAAA GAAGCTGCTC GACTAGCAAA GGAAAAATCA
CAAGAGAAGA TGGAACTTAC CAGTACACCT TCACAGGAAT CCGCAGGCGG GGATCTTCAG
TCTCCTTTAA CACCAGAAAG TA


MGEQPIFSTR AHVFQIDPNT KKNWVPTSKH AVTVSYFYDS TRNVYRIISL DGSKAIINST
ITPNMTPTKT SQKFGQWADS RANTVYGLGF SSEHHLSKFA EKFQEFKEAA RLAKEKSQEK
MELTSTPSQE SAGGDLQSPL TPES

.


Appendix 3. Homer gene sequence amplified from rat astrocyte mRNA and
its corresponding amino acid sequence

ATGGGGGA ACAACCTATC TTCAGCACTC GAGCTCATGT CTTCCAGATC GACCCAAACA
CAAAGAAGAA CTGGGTACCC ACCAGCAAGC ATGCAGTTAC TGTGTCTTAT TTCTATGACA
GCACAAGGAA TGTGTATAGG ATAATCAGTC TAGACGGCTC AAAGGCAATA ATAAATAGCA
CCATCACTCC AAACATGACA TTTACTAAAA CATCTCAAAA GTTTGGCCAA TGGGCTGATA
GCCGGGCAAA CACTGTTTAT GGACTGGGAT CTCCTCTGA GCATCATCTC TCAAAATTTG
CAGAAAAGTT TCAGGAATTT AAAGAAGCTG CTCGGCTGGC AAAGGAGAAG TCGCAGGAGA
AGATGGAACT GACCAGTACC CCTTCACAGG AATCAGCAGG AGGAGATCTT CAGTCTCCTT
TAACACCAGA


MGEQPIFSTR AHVFQIDPNT KKNWVPTSKH AVTVSYFYDS TRNVYRIISL DGSKAIINST
ITPNMTPTKT SQKFGQWADS RANTVYGLGF SSEHHLSKFA EKFQEFKEAA RLAKEKSQEK
MELTSTPSQE SAGGDLQSPL TP

Appendix 4 Homer gene sequence amplified from CHO cells mRNA and its corresponding amino acid sequence

```
TTCAGCACTC GAGCTCATGT CTTCCAGATT GACCCAAACA CAAAGAAGAA CTGGGTACCC
ACCAGCAAGC ATGCAGTTAC TGTATCTTAT TTTTATGACA GCACAAGAAA TGTATATAGG
ATAATCAGTT TAGATGGCTC AAAGGCAATA ATAAATAGCA CCATCACTCC AAACATGACA
TTTACTAAAA CATCTCAAAA GTTTGGCCAG TGGGCTGATA GCCGGGCAAA TACTGTTTAT
GGATTGGGAT TCTCCTCTGA GCATCATCTT TCCAAATTTG CAGAAAAGTT TCAGGAATTT
AAAGAAGCTG CTCGTCTTGC AAAGGAGAAG TCACAGGAGA AGATGGAACT GACCAGTACA
CCTTCACAGG AATCAGCAGG TGGAGATCTT CAGTCTCCTT TAACACCGAA AGGT
```

```
FSTRAHVFQI DPNTKKNWVP TSKHAVTVSY FYDSTRNVYR IISLDGSKAI INSTITPNMT
FTKTSQKFGQ WADSRANTVY GLGFSSEHHL SKFAEKFQEF KEAARLAKEK SQEKMELTST
PSQESAGGDL QSPLTPKG
```

| | | | | | | |
|---|---|---|---|---|---|---|
| Ebadi M. And Srinivasan S.K. | 1995 | Pharmacological Reviews 47: | 575–604 | | | |
| Brakeman P.R., Lanahan A.A., O'Brien R., Roche K., Barnes C.A., Huganir R.L., and Worley P.F. | | | | 1997 | Nature 386: | 284–288 |
| Ozawa K.A., Saltoh F., Hirai K., Inokuchi K. | 1997 | FEBS Letters 412: | 183–189 | | | |
| Wright I. And Woodruff P. | 1995 | CNS Drugs 3: | 126–144 | | | |
| Köpfel T. And Gasparinin F. | 1996 | Drug Development Today 1: | 103–108 | | | |
| Ponting C.P. And Phillips C. | 1995 | Trends in Biochemistry 20: | 102–103 | | | |
| Reynolds G.P. And Czudek C. | | | 461–503 | | | |
| Gratz S.S. And Simpson G.M. | 1994 | CNS Drugs 2: | 429–439 | | | |
| Seeman P. | 1987 | Synapse 1: | 133–152 | | | |
| Fatemi H., Meltzer H.Y. And Roth B.L. | 1996 | In Anti–psychotics Ed. Csernansky J.G. | 77–115 | | | |
| Deutch A.Y. | 1996 | In Anti–psychotics Ed. Csernansky J.G. | 117–161 | | | |
| Garcia–Ladona, F.J., Palacios, J.M., and Mengod G.. | 1994 | Molecular Brain Research 21: | 75–84 | | | |
| Garcia–Ladona F.J., Huss Y., Frey P.,and Ghandour M.S. | 1997 | J. Neuroscience Research 50: | 50–61 | | | |
| Garcia–Ladona F.J., Claro, E., Garcia, A., and Picatoste, F. | 1993 | Neuroreport 4: | 691–694 | | | |
| Garcia–Ladona F.J., De Barry, J., Girard, C., and Gombos G. (1991) Ectopic granule cell layer in | 1991 | Exp. Brain Research 86: | 90–96. | | | |
| Lipska B.K., Jaskiw G.E., and Weinberger D.R. | 1993 | Neuropsychopharmacology 9: | 67–75 | | | |
| Swerdlow N.R., Braff D.L., Bakshi V.P., and Geyer M.A. | 1996 | In Anti–psychotics Ed. Csernansky J.G. | 289–307 | | | |
| J.T. August | 1997 | Gene Therapy in Advances in Pharmacology | 40 Academic Press | | | |
| H.G. Vogel and W.H. Vogel Eds. | 1997 | Drug Discovery and Evaluation | | | | |

**Claims**

1. A method for treatment of psychosis in a human being comprising administering to said human being a composition comprising an effective amount of a homer expression inducing compound.

2. A method for treatment of psychosis in a human being comprising administering to said human being a composition comprising an effective amount of a homer expression inhibiting compound.

3. A method for treatment of schizophrenia in a human being comprising administering to said human being a composition comprising an effective amount of a homer expression inducing compound.

4. A method for treatment of schizophrenia in a human being comprising administering to said human being a composition comprising an effective amount of a homer expression inhibiting compound.

5. A method for treatment of oncological disorders in a human being comprising administering to said human being a composition comprising an effective amount of a homer expression inhibiting compound.

6. A method for treatment of oncological disorders in a human being comprising administering to said human being a composition comprising an effective amount of a homer expression inducing compound.

7. A method for treatment of neuroleptical disorders in a human being comprising administering to said human being a composition comprising an effective amount of a compound which interacts with metabotropic receptors.

8. A method for treatment of neuroleptic maligne syndrome in a human being comprising administering to said human being a composition comprising an effective amount of a compound which interacts with metabotropic receptors and/or homer.

9. A method for treatment of illnesses in a human being comprising administering to said human being a composition comprising an effective amount of a compound which is able to interfere with homer and its interaction with metabotropic and/or sigma receptors.

10. An isolated nucleic acid according to the sequences as can be seen from appendix 1 to 4.

11. A polypeptide sequence encode by the sequences of claim 7.

12. A method of screening for new compounds as therapeutics using astrocytes, Hel, A-172, U97 and/or glial cells expressing homer.

13. A method of treatment of CNS disorders in a human being via glial cells comprising administering to said human being a composition comprising an effective amount of a compound, which is able to act on glial cells and which is able to inhibite the expression of homer.

14. A method of treatment of CNS disorders in a human being comprising administering to said human being a composition comprising an effective amount of a compound, which is able to act on glial cells and which is able to inhibite the expression of homer.

15. A method of treatment of CNS disorders in a human being comprising administering to said human being a composition comprising an effective amount of a compound, which is able to act on glial cells and which is able to activate the expression of homer.

16. A method of treatment of CNS disordersin a human being comprising administering to said human being a composition comprising an effective amount of a compound, which is able to act on astrocytes and which is able to inhibite the expression of homer.

17. A method of treatment of CNS disorders in a human being comprising administering to said human being a composition comprising an effective amount of a compound, which is able to act on astrocytes and which is able to activate the expression of homer.

Fig. 1

Fig. 2c

Homer mRNA levels in cortical regions of rat brain
Dose-effect experiment

Fig. 2b

Homer mRNA levels in striatal regions of rat brain
Dose-effect experiment

Homer mRNA levels in limbic regions of rat brain
Dose-effect experiment

Fig. 2a